Europäisches Patentamt

European Patent Office

Office européen des brevets

(11) Numéro de publication : **0 359 628 B1**

# (12) FASCICULE DE BREVET EUROPEEN

(45) Date de publication du fascicule du brevet :
**12.08.92 Bulletin 92/33**

(51) Int. Cl.$^5$ : **B01D 53/22, C01B 3/50**

(21) Numéro de dépôt : **89402432.2**

(22) Date de dépôt : **07.09.89**

(54) **Procédé et installation de récupération des hydrocarbures les plus lourds d'un melange gazeux.**

(30) Priorité : **12.09.88 FR 8811882**

(43) Date de publication de la demande :
**21.03.90 Bulletin 90/12**

(45) Mention de la délivrance du brevet :
**12.08.92 Bulletin 92/33**

(84) Etats contractants désignés :
**AT BE CH DE ES FR GB GR IT LI LU NL SE**

(56) Documents cités :
**EP-A- 0 100 923**
**EP-A- 0 178 172**
**EP-A- 0 213 525**
**EP-A- 0 266 271**
**DE-A- 2 947 239**
**US-A- 4 732 583**

(73) Titulaire : **L'AIR LIQUIDE, SOCIETE ANONYME POUR L'ETUDE ET L'EXPLOITATION DES PROCEDES GEORGES CLAUDE**
**75, Quai d'Orsay**
**F-75321 Paris Cédex 07 (FR)**

(72) Inventeur : **Gauthier, Pierre**
**95 bis, bd Jean Jaurès**
**F-94260 Fresnes (FR)**
Inventeur : **Monereau, Christian**
**159, rue de Charonne**
**F-75011 Paris (FR)**

(74) Mandataire : **Le Moenner, Gabriel et al**
**L'AIR LIQUIDE 75, Quai d'Orsay**
**F-75321 Paris Cédex 07 (FR)**

## Description

La présente invention est relative à la technique de récupération des fractions C2+ ou C3+ d'un mélange gazeux d'hydrocarbures C1-C5 riche en hydrogène. Elle s'applique en particulier à la récupération des fractions C2+ ou C3+ d'un gaz résiduaire de raffinerie.

Dans la EP-A-0 178 172 est décrit un procédé pour la récupération de l'hydrogène et de différentes fractions d'hydrocarbures à partir d'un courant gazeux provenant d'un procédé de conversion d'hydrocarbures accompagné d'une formation d'hydrogène. L'hydrogène est séparé par perméation et le procédé comprend en outre des étapes d'adsorption, de condensation et de distillation.

Dans le brevet US-A-4 732 583 est décrit un procédé pour la purification de l'hydrogène et sa séparation des hydrocarbures contenu dans des gaz résiduaires de raffinerie. L'hydrogène séparé du mélange gazeux par perméation et condensation partielle est ensuite redirigé vers le procédé dont il provient.

Dans la EP-A-0 213 525 est décrit un procédé similaire. Le courant gazeux riche en hydrogène séparé dans l'étape de condensation et recyclé vers le coté haute pression du perméateur.

Dans la EP-A-0 266 271 est décrit un procédé de séparation de gaz par perméation, l'espace basse pression du perméateur étant balayé par un gaz contenant au moins un des gaz à retenir du coté haute pression pour ainsi augmenter le flux du gaz à séparer à travers la membrane.

Suivant des procédés classiques de récupération, on épure le mélange, notamment par adsorption, en en éliminant les impuretés telles que H2O, HCl, NH3,..., puis on condense partiellement le mélange épuré, puis on soumet le condensat à une distillation. Ces procédés sont relativement coûteux en investissement et en énergie car ils conduisent à traiter par condensation puis distillation la quasi-totalité du mélange de départ, lequel contient une proportion très importante de gaz légers qu'il faut éliminer, par exemple 60 à 90 % d'hydrogène ainsi que du méthane.

L'invention a pour but de perfectionner cette technique de façon à la rendre plus économique.

A cet effet, l'invention a pour objet un procédé du type précité, caractérisé en ce qu'on élimine la majeure partie de l'hydrogène par perméation en amont de l'étape de condensation, et en ce qu'on dilue le perméat dans le perméateur, au moyen de gaz résiduaire de l'étape de distillation.

L'invention a également pour objet une installation destinée à la mise en oeuvre d'un tel procédé. Cette installation, du type comprenant un appareil d'épuration, notamment par adsorption, des impuretés telles que H2O, HCL, NH3,..., un échangeur de chaleur indirect relié à la sortie de cet appareil et associé à son bout froid à un séparateur de phases, et une colonne de distillation reliée à la partie inférieure du séparateur de phases, est caractérisée en ce qu'elle comprend un perméateur en amont de l'échangeur, et des moyens pour introduire dans l'espace basse pression du perméateur du gaz résiduaire de la colonne de distillation.

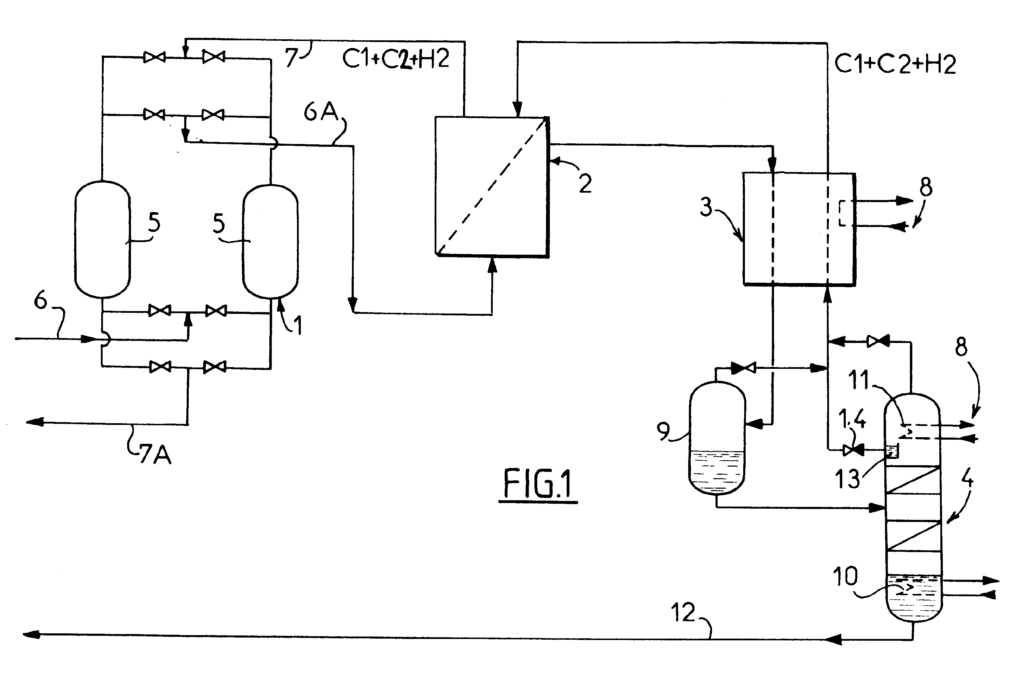

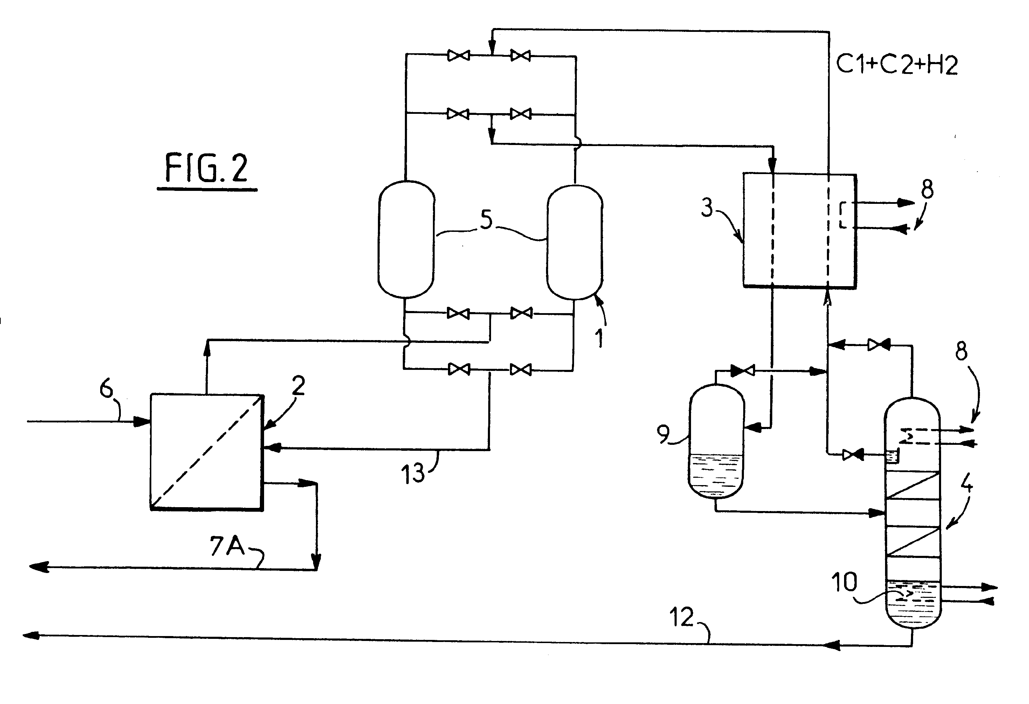

Quelques exemples de mise en oeuvre de l'invention vont maintenant être décrits en regard des dessins annexés, sur lesquels les figures 1 et 2 représentent schématiquement plusieurs variantes de l'installation suivant l'invention.

L'installation représentée à la figure 1 comprend essentiellement un appareil d'épuration 1, un perméateur 2, un échangeur de chaleur 3 et une colonne de distillation 4. Cette installation est destinée à récupérer les gaz de pétrole liquéfiés (GPL, constitués par les fractions C3+, c'est-à-dire par un mélange de propane, de butane et de pentane) contenus dans un gaz résiduaire de raffinerie, tel qu'un gaz de "platforming" contenant en outre 60 à 90 % d'hydrogène, du méthane, de l'éthane et diverses impuretés telles que N2, CO2, H2S, HCL,H2O, NH3 et CO.

L'appareil 1 comprend deux bouteilles d'adsorption 5 à fonctionnement alterné, l'une étant en phase d'adsorption pendant que l'autre est en phase de régénération ou désorption à contre-courant. L'adsorbant contenu dans les bouteilles 5 est par exemple du tamis moléculaire. Cet appareil 1 est alimenté par une conduite 6 véhiculant le mélange à traiter, et par une conduite 7 véhiculant le gaz de régénération. Il comprend un ensemble de vannes approprié pour le fonctionnement qui sera décrit plus loin.

Le perméateur 2 est adapté pour séparer l'hydrogène des autres constituants du mélange, par exemple grâce à un faisceau de fibres creuses constituées par une membrane à perméabilité sélective. Un exemple de membrane convenant pour cette application est basé sur une technologie polyaramide développée par DU PONT de NEMOURS selon le US-E-30351 (re-issue de US-A-3 899 309). D'autres exemples sont décrits dans les brevets US-A-4 180 553 et US-A-4 230 463.

L'échangeur de chaleur 3 est du type indirect à circulation à contre-courant des fluides mis en relation d'échange thermique. Il comprend une partie chaude refroidie par un groupe frigorifique 8 fonctionnant vers -30 à -40°C et est associé à un séparateur liquide-vapeur 9 disposé à son bout froid.

La colonne de distillation 4 fonctionne en déméthaniseur-dééthaniseur et comporte en cuve un rebouilleur 10 dont la température est comprise entre 40 et 100°C et en tête un condenseur 11 qui peut être refroidi par le groupe frigorifique 8 précité.

En fonctionnement, le gaz à traiter arrive à une pression comprise entre 15 et 30 bars, à une température voisine de l'ambiante, par la conduite 6 et est épuré, c'est-à-dire débarrassé des impuretés telles que $H_2O$, $NH_3$, HCL,..., par l'appareil 1. Il passe ensuite via une conduite 6A dans l'espace haute pression du perméateur 2, où il est débarrassé de la majeure partie de l'hydrogène qu'il contient. Ainsi, c'est essentiellement un mélange d'hydrocarbures qui pénètre dans l'échangeur 3 et en ressort partiellement condensé à une température comprise entre -60 et -90°C pour être séparé dans le séparateur 9 en deux phases, à savoir une phase vapeur constituée essentiellement de méthane et d'éthane et contenant le reliquat d'hydrogène, et un condensat constitué essentiellement de C2+ et contenant le reliquat de méthane. Le condensat recueilli dans le séparateur 9 est envoyé à mi-hauteur dans la colonne 4, laquelle produit en cuve les GPL désirés, évacués par une conduite 12. La condensation partielle ci-dessus décrite peut s'effectuer en une ou plusieurs étapes, avec des séparations de condensats successives, conformément à la pratique industrielle classique.

Une partie du liquide condensé en tête de la colonne 4 est soutiré en 13 et détendu dans une vanne de détente 14, après ou sans sous-refroidissement dans l'échangeur 3, puis est mélangé à la vapeur de tête de la colonne (méthane et éthane) et à celle séparée dans le séparateur 9, ces deux vapeurs étant toutes deux plus légères que le liquide en question et étant détendues à la même pression. Le mélange traverse l'échangeur 3 à contre-courant du gaz à traiter, et sa vaporisation et son réchauffement à une pression comprise entre 3 et 5 bars permettent de refroidir le gaz à traiter jusqu'à la température froide précitée.

Ce mélange, après réchauffage dans l'échangeur 3, est introduit dans l'espace basse pression du perméateur 2 pour y être mélangé avec l'hydrogène basse pression constituant le perméat, et l'ensemble est ensuite utilisé pour régénérer l'adsorbant de l'appareil 1, via la conduite 7, puis constitue un résiduaire de l'installation pouvant servir de "fuel gas" et évacué par une conduite 7A.

Grâce à l'élimination de d'hydrogène par le perméateur 2 en amont de la partie cryogénique 3-4 de l'installation, l'investissement et la dépense d'énergie concernant cette partie de l'installation sont réduits. De plus, la dilution du perméat décrite ci-dessus a pour effet d'abaisser la pression partielle d'hydrogène dans l'espace basse pression du perméateur et permet par suite soit d'augmenter la pression du résiduaire de l'installation (fuel gas), soit d'aumenter l'efficacité de la perméation.

En variante, comme représenté sur la figure 2, la séparation de l'hydrogène par perméation peut s'effectuer en amont de l'appareil 1, ce qui permet de réduire l'investissement relatif à l'unité d'épuration, d'autant plus que les membranes perméables à l'hydrogène laissent passer en même temps que l'hydrogène une partie non négligeable des impuretés du mélange. La dilution du perméat s'effectue alors de préférence au moyen du gaz ayant effectué la régénération de l'adsorbant, via une conduite 13.

On comprend que la modification des paramètres du procédé permet d'appliquer l'invention à la récupération non seulement des GPL, mais également de l'éthane. Par ailleurs, l'invention s'applique aux cas où la membrane du perméateur laisse passer des quantités substantielles non seulement d'hydrogène, mais également des autres constituants légers CH4 et éventuellement C2H6.

**Revendications**

1. Procédé de récupération des fractions C2+ et C3+ d'un mélange gazeux d'hydrocarbures C1 - C5 riche en hydrogène, notamment d'un gaz résiduaire de raffinerie, du type dans lequel on épure le mélange, notamment par adsorption (en 1), en en éliminant les impuretés telles que $H_2O$, HCL, $NH_3$,..., puis on condense partiellement le mélange épuré (en 3), puis on soumet le condensat à une distillation (en 4) dont on retire les fractions C2+ ou C3+ comme produits en cuve, caractérisé en ce qu'on élimine la majeure partie de l'hydrogène par perméation (en 2) en amont de l'étape de condensation, en ce qu'on dilue le perméat dans le perméateur (2), au moyen de gaz résiduaire de l'étape de distillation

2. Procédé suivant la revendication 1, caractérisé en ce qu'on effectue la perméation (en 2) entre l'étape d'épuration (en 1) et l'étape de condensation (en 3).

3. Procédé suivant la revendication 1, caractérisé en ce qu'on effectue la perméation (en 2) en amont de l'étape d'épuration.

4. Procédé suivant l'une quelconque des revendications 1 à 3, caractérisé en ce qu'on soutire (en 13) une partie du liquide condensé en tête de la colonne de distillation (4), on détend (en 14) le liquide soutiré, et l'on vaporise le liquide détendu à contre-courant du gaz à traiter, en relation d'échange thermique avec celui-ci, pendant l'étape de condensation.

5. Procédé suivant la revendication 4, caractérisé en ce qu'on ajoute au liquide détendu, avant sa vaporisation, du gaz issu de l'étape de condensation et/ou du gaz résiduaire de l'étape de distillation.

6. Installation pour la mise en oeuvre du procédé selon la revendication 1, du type comprenant un appareil (1) d'épuration, notamment par adsorption, des impuretés telles que $H_2O$, HCL, $NH_3$,..., un échangeur de chaleur indirect (3) relié à la sortie de cet appareil et associé à son bout froid à un séparateur de phases (9), et une colonne de distillation (4) reliée à la partie inférieure du séparateur de phases, caractérisée en ce qu'elle comprend un perméateur (2) en amont de l'échangeur de chaleur (3), et des moyens pour introduire dans l'espace basse pression du perméateur (2) du gaz résiduaire de la colonne de distillation (4).

7. Installation suivant la revendication 6, caractérisée en ce que le perméateur (2) est interposé entre l'appareil d'épuration (1) et l'échangeur de chaleur (3).

8. Installation suivant la revendication 6, caractérisée en ce que le perméateur (2) est disposé en amont de l'appareil d'épuration (1).

9. Installation suivant l'une quelconque des revendications 6 à 8, caractérisée en ce que la colonne de distillation (4) comporte des moyens (13) pour soutirer une partie du liquide condensé en tête de colonne, des moyens (14) pour détendre le liquide soutiré, et des moyens pour faire passer le liquide détendu dans l'échangeur de chaleur (3).

10. Installation suivant la revendication 9, caractérisée en ce qu'elle comprend des moyens pour ajouter au liquide détendu, au bout froid de l'échangeur (3), du gaz issu du séparateur de phases (9) et/ou du gaz de tête de la colonne de distillation (4).

**Patentansprüche**

1. Verfahren zur Gewinnung von C2+- und C3+-Fraktionen eines an Wasserstoff reichen gasförmigen C1 - C5-Kohlenwasserstoffgemisches, insbesondere eines Raffinerierestgases, bei dem man das Gemisch, insbesondere durch Adsorption (bei 1) reinigt, wobei man die Verunreinigungen, wie $H_2O$, HCl, $NH_3$ ... beseitigt, dann das gereinigte Gemisch (bei 3) teilweise kondensiert und dann das Kondensat einer Destillation (bei 4) unterzieht, bei der man die C2+- oder C3+-Fraktionen als Bodenprodukt abzieht, dadurch gekennzeichnet, daß man den größeren Teil des Wasserstoffes durch Permeation (bei 2) aufstromwärts von der Kondensationsstufe beseitigt und daß man das Permeat in der Permeationsvorrichtung (2) mit Restgas der Destillationsstufe verdünnt.

2. Verfahren nach Anspruch 1, dadurch gekennzeichnet, daß man die Permeation (bei 2) zwischen der Reinigungsstufe (bei 1) und der Kondensationsstufe (bei 3) durchführt.

3. Verfahren nach Anspruch 1, dadurch gekennzeichnet, daß man die Permeation (bei 2) aufstromwärts von der Reinigungsstufe durchführt.

4. Verfahren nach einem der Ansprüche 1 bis 3, dadurch gekennzeichnet, daß man einen Teil der am Kopf der Destillationskolonne (4) kondensierten Flüssigkeit (bei 13) abnimmt, daß man die abgenommene Flüssigkeit (bei 14) entspannt und daß man die entspannte Flüssigkeit im Gegenstrom des zu behandelnden Gases in Wärmeaustauschbeziehung mit diesem während der Kondensationsstufe verdampft.

5. Verfahren nach Anspruch 4, dadurch gekennzeichnet, daß man zu der entspannten Flüssigkeit vor ihrer Verdampfung aus der Kondensationsstufe stammendes Gas und/oder Restgas der Destillationsstufe zusetzt.

6. Vorrichtung zur Durchführung des Verfahrens nach Anspruch 1 mit einer Einrichtung (1) zur Entfernung, insbesondere durch Adsorption, von Verunreinigungen, wie $H_2O$, HCl, $NH_3$ ..., einem indirekten Wärmetauscher (3), der mit dem Ausgang dieser Einrichtung verbunden und an seinem kalten Ende mit einer Phasentrenneinrichtung (9) verbunden ist, und einer Destillationskolonne (4), die mit dem unteren Teil der Phasentrenneinrichtung verbunden ist, dadurch gekennzeichnet, daß sie eine Permeationseinrichtung (2) aufstromwärts von dem Wärmetauscher (3) und Einrichtungen zur Einführung von Resedas der Destillationskolonne (4) in den Niederdruckraum der Permeationseinrichtung (2) umfaßt.

7. Vorrichtung nach Anspruch 6, dadurch gekennzeichnet, daß die Permeationseinrichtung (2) zwischen die Reinigungseinrichtung (1) und den Wärmetauscher (3) eingeschaltet ist.

8. Vorrichtung nach Anspruch 6, dadurch gekennzeichnet, daß die Permeationseinrichtung (2) aufstromwärts von der Reinigungseinrichtung (1) angeordnet ist.

9. Vorrichtung nach einem der Ansprüche 6 bis 8, dadurch gekennzeichnet, daß die Destillationskolonne (4) Einrichtungen (13) zum Abnehmen eines Teils der am Kopf der Kolonne kondensierten Flüssigkeit, Einrichtungen (14) zum Entspannen der abgenommenen Flüssigkeit und Einrichtungen, um die entspannte Flüssigkeit in den Wärmetauscher (3) gehen zu lassen, aufweist.

10. Vorrichtung nach Anspruch 9, dadurch gekennzeichnet, daß sie Einrichtung zum Zugeben von aus der Phasentrenneinrichtung (9) stammendem Gas und/oder von Kopfgas der Destillationskolonne (4) zu der entspannten Flüssigkeit am kalten Ende des Tauschers (3) umfaßt.

**Claims**

1. Process for recovering fractions C2+ and C3+ from a gaseous mixture of hydrocarbons C1 - C5 which is rich in hydrogen, in particular a residual refinery gas, of the type in which the mixture is purified, in particular by adsorption (at 1), by eliminating therefrom impurities such as $H_2O$, HCl, $NH_3$,..., then the purified mixture is partly condensed (at 3), then the condensate is subjected to distillation (at 4), from which the fractions C2+ or C3+ are withdrawn like products in a tank, characterised in that the greater part of the hydrogen is eliminated by permeation (at 2) upstream of the condensation stage, in that the permeate is diluted in the permeating arrangement (2) by means of residual gas of the distillation stage.

2. Process according to claim 1, characterised in that the permeation (at 2) is carried out between the purification stage (at 1) and the condensation stage (at 3).

3. Process according to claim 1, characterised in that the permeation (at 2) is carried out upstream of the purification stage.

4. Process according to any one of the claims 1 to 3, characterised in that a portion of the condensed liquid is drawn off (at 13) at the top of the distillation column (4), the pressure on the liquid which has been drawn off is decreased (at 14) and the liquid, the pressure on which has been decreased, is vaporized in counter-current to the gas to be treated, in relation to thermal exchange with the latter, during the condensation stage.

5. Process according to claim 4, characterised in that there is added to the liquid, the pressure on which has been decreased, before its vaporization, gas which is derived from the condensation stage and/or the residual gas of the distillation stage.

6. Installation for implementing the process according to claim 1, of the type which comprises an apparatus (1) for purification, in particular by adsorption, of the impurities such as $H_2O$, $HCl$, $NH_3$,..., an indirect heat exchanger (3) connected to the outlet of this apparatus and associated at its cold end with a phase separator (9), and a distillation column (4) connected to the lower part of the phase separator, characterised in that it comprises a permeating arrangement (2) upstream of the heat exchanger (3) and means for introducing into the low-pressure space of the permeating arrangement (2) residual gas of the distillation column (4).

7. Installation according to claim 6, characterised in that the permeating arrangement (2) is interposed between the purifying apparatus (1) and the heat exchanger (3).

8. Installation according to claim 6, characterised in that the permeating arrangement (2) is arranged upstream of the purifying apparatus (1).

9. Installation according to any one of the claims 6 to 8, characterised in that the distillation column (4) comprises means (13) for drawing off a portion of the condensed liquid at the top of the column, means (14) for decreasing the pressure on the liquid which has been drawn off and means for allowing the liquid, the pressure on which has been decreased, to pass into the heat exchanger (3).

10. Installation according to claim 9, characterised in that it comprises means for adding to the liquid, the pressure on which has been decreased, at the cold end of the exchanger (3), gas which is derived from the phase separator (9) and/or gas from the top of the distillation column (4).

FIG.1

FIG.2

C1+C2+H2

EP 0 359 628 B1